# EUROPÄISCHE PATENTSCHRIFT

(11) **EP 0 561 183 B1**
(45) Veröffentlichungstag und Bekanntmachung des Hinweises auf die Patenterteilung: **19.08.1998**
(21) Anmeldenummer: 93102895.5
(22) Anmeldetag: 25.02.1993
(51) Int. Cl.: C12P 21/08, A61K 39/395, C07K 2/00

(54) **Monoklonaler Anti-Gangliosid-Antikörper, seine Herstellung und Verwendung als Tumortherapeutikum**
Anti-ganglioside monoclonal antibody, its preparation and use as a tumour therapeutic agent
Anticorps monoclonal contre les gangliosides, sa préparation et son utilisation comme agent thérapeutique antitumoral

(30) Priorität: 19.03.1992 DE 4208795
(43) Veröffentlichungstag der Anmeldung: 22.09.1993
(73) Patentinhaber: BEHRINGWERKE Aktiengesellschaft, 35001 Marburg (DE)
(72) Erfinder: Bosslet, Klaus, W-3550 Marburg (DE); Seemann, Gerhard, W-3550 Marburg-Elnhausen (DE); Dippold, Wolfgang, W-6500 Mainz (DE)
(74) Vertreter: Fischer, Hans-Jürgen, Dr.

(56) Entgegenhaltungen:
- EP-A- 189 849
- EP-A- 234 122
- EP-A- 316 882
- EP-A- 351 731
- EP-A- 493 686
- US-A- 5 055 559
- JOURNAL OF NEUROCHEMISTRY Bd. 54, Nr. 2, Februar 1990, US Seiten 513 - 517 H. YAMAMOTO ET AL. 'Tetrasialoganglioside GQ1b reactive monoclonal antibodies: their characterization and application for quantification of GQ1b in some cell lines of neuronal and adrenal origin(s)'
- BIOCHIM. BIOPHYS. ACTA Bd. 1123, Nr. 2, 24. Januar 1992, AMSTERDAM, NL Seiten 184 - 190 H. OZAWA ET AL. 'Generation of one set of monoclonal antibodies specific for b-pathway ganglio-series gangliosides'
- CANCER RESEARCH Bd. 44, Nr. 2, Februar 1984, US Seiten 806 - 810 W.G. DIPPOLD ET AL. 'Inhibition of human melanoma cell growth in vitro by monoclonal anti- GD3-ganglioside antibody'
- EUR. J. CANCER Bd. 28A, Nr. 10, 1992, Seiten 1605 - 1610 W. DIPPOLD ET AL. 'Immunorecognition of ganglioside epitopes: correlation between affinity and cytotoxicity of ganglioside antibodies'
- CANCER IMMUNOLY IMMUNOTHERAPY Bd. 36, Nr. 4, 1993, GERMANY Seiten 260 - 266 S. OHTA ET AL. 'Antitumor effects of a novel monoclonal antibody with high binding affinity to ganglioside GD3'

## Beschreibung

Murine monoklonale Antikörper (MAk), die spezifisch für tumorassoziierte Antigene sind, werden sowohl in der in vitro Diagnostik im Rahmen von Tumormarkertests als auch in der in vivo Diagnose von Tumoren im Rahmen der Immunszintigraphie verwendet. Trotz vieler Versuche, mit MAk tumortherapeutische Effekte zu erzielen, waren die klinisch-therapeutischen Befunde in vielen Fällen statistisch nicht signifikant. Ursachen für diese Mißerfolge liegen sowohl in der unzureichenden Penetration der Tumore durch die MAk, ihrer Immunogenität sowie ihrem niedrigen zytotoxischen Potential und ihrer Kreuzreaktivität mit bestimmten Normalgeweben.

Die unzureichende Penetration menschlicher Tumore kann durch wiederholte Langzeitgabe hoher MAk-Dosen überwunden werden. Dies ist allerdings nur möglich, wenn die MAk wenig immunogen sind. Solche wenig immunogenen Moleküle lassen sich durch Humanisierung (Güssow, D., Seemann G., Methods in Enzymology, pp. 203, 1991) von MAk herstellen (huMAk). HuMAk besitzen, wenn sie mit einem entsprechenden Fc-Teil versehen sind, ein höheres zytotoxisches Potential. Wenn der MAk nun noch eine hohe Tumorspezifität und hohe Affinität besitzt, sollte ein hoch wirksames Tumortherapeutikum entwickelbar sein.

Bei Versuchen, hochaffine MAk gegen Ganglioside herzustellen, stellten wir fest, daß die MAk im allgemeinen niedrige Affinität besaßen und signifikant mit relevanten menschlichen Normalgeweben kreuzreagierten.

Yamamoto, H. et al. (J. Neurochem. 54: 513-517, 1990) haben die monoklonalen Antikörper 1H10, 2C7 und 3F4 erzeugt, die besonders stark mit dem Gangliosid GQ1b reagieren. Alle drei Antikörper sind vom IgM-Isotyp, der für immunologische Techniken weniger geeignet ist. Ziel von Ozawa, H. et al. (Biochem. Biophys. Acta 1123: 184-190, 1992) war die Erzeugung von Antikörpem gegen eine Familie von Gangliosiden inklusive GD3 und GQ1b. Dabei wurde der dem MAk R24 (EP-A-0189849) sehr ähnliche MAk GGB19 generiert. Der MAk R24 unterscheidet sich jedoch in wesentlichen Eigenschaften von den im folgenden näher beschriebenen erfindungsgemäßen Antikörpern.

Überraschenderweise ist es uns jedoch gelungen, einen hochaviden MAk zu erzeugen, der mit den Gangliosiden GD3 und GQ1b reagiert, bei niedrigen molaren Konzentrationen bereits hohe zytotoxische Aktivität gegenüber Melanomzellen entfaltet, aber minimale Kreuzreaktivität mit menschlichen Normalgeweben zeigt. Die Ganglioside GD3 und GQ1b sind strukturell eng verwandte Ganglioside. Das Hybridom 2121, welches den MAk BW 2121 ausscheidet, wurde am 05.03.1992 bei der DSM - Deutsche Sammlung von Mikroorganismen und Zellkulturen GmbH, Mascheroder Weg 1 B, 3300 Braunschweig, gemäß dem Budapester Vertrag unter der Hinterlegungsnummer DSM ACC 2036 hinterlegt.

Das Hybridom 2121 kann wie folgt hergestellt werden:

Immunkompetente Säugetiere, wie beispielsweise die Maus, Ratte oder das Schaf, werden mit GD3- und/oder GQ1b-haltigem Immunogen, z.B. Melanomzellen oder Extrakten hieraus, immunisiert, die Immunzellen durch Fusion mit Myelomzellen immortalisiert und die entstehenden Hybridomaklone auf Ausscheidung von GD3 und GQ1b spezifischen MAk getestet. Alternativ kann mittels gentechnischer Methoden aus den Immunzellen immunisierter Säugetiere eine Gen-Bank angelegt werden, die die Gene, welche für die variablen Bereiche der Antikörper kodieren, enthält und aus dieser Genbank können dann beispielsweise über Phagenscreeningtechniken die Klone mit der gewünschten Mak-Spezifität isoliert und kloniert werden.

Gegenstand der Erfindung ist daher:
Das Hybridom 2121 (DSM ACC 2036)

Ein monoklonaler Antikörper (BW 2121), der von dem Hybridom 2121 abstammt.

Monoklonale Antikörper oder Teile davon, die an ein Epitop, das von einem monoklonalen Antikörper des Hybridoms 2121 erkannt wird, binden und immunhistochemisch nicht mit der Medulla der Nebenniere sowie mit den Goormaghtigh Zellen reagieren.

Ferner gehören zum Gegenstand der Erfindung monoklonale Antikörper oder Teile davon, die chimärer, humanisierter, bi- oder oligospezifischer Art sind.

Humanisierte Antikörper sind besonders bevorzugte Beispiele davon.

Darüberhinaus sind Gegenstand der Erfindung ein Verfahren zur Herstellung der erfindungsgemäßen Antikörper, Verfahren zum Nachweis von Melanomen und anderen GD3 und GQlb exprimierenden Tumoren oder Geweben unter Verwendung der erfindungsgemäßen Antikörper und eine entsprechende pharmazeutische Zusammensetzung bzw. ein Diagnostikum.

Der MAk BW 2121 kann auch durch folgende 7 Merkmale definiert werden:
1) Im ELISA und Magnani ITLC-Test (Magnani, J.L., Brockhaus, M., Smith, D.F., Ginsburg, V., Science 212: 55-57, 1982) reagiert der MAk mit Gangliosiden, die nach der Nomenklatur von Svennerholm (J. Neurochem. 10: 613-623, 1963) als GD3 und GQ1b bezeichnet sind. Er bindet nicht an GD1b, GD2, GT1b, GD1a, GM1, GM2 und GM3.
2) Aus einer Reihe von 10 kryopräservierten Melanom-Primärtumoren bindet der MAk BW 2121 nach immunhistochemischer Färbung (Dippold, W.G., Dienes, H.P., Knuth, A., Meyer zum Büschenfelde, K.-H., Cancer Res. 45: 3699-3705, 1985) homogen an 6 von 10 Tumore, d.h. > 90 % der Melanomzellen sind gleichmäßig gefärbt, 3 von 10 Tumoren reagieren weniger stark, d.h. 10-50 % der Melanomzellen zeigen eine deutlich positive Reaktion. 1 von 10 Melanom Primärtumoren reagierte nicht mit dem MAk.
   Ein ähnliches Reaktionsprofil, allerdings wesentlich heterogener, ergibt sich bei den untersuchten 14 Melanommetastasen. Hier reagiert der MAk BW 2121 mit > 90% der Tumorzellen bei 6 von 14 Tumoren. Bei 5 von 14 Metastasen waren zwischen 5 und 50 % der Tumorzellen positiv, wohingegen 3 Metastasen überhaupt keine Reaktion zeigten.
3) Eine immunhistochemische Untersuchung naevozellulärer Naevi ergab folgendes Ergebnis:
   MAk BW 2121 reagierte mit 3 von 4 Verbindungsnaevi (junctional nevi), mit 13 von 14 zusammengesetzen Naevi (compound nevi), 3 von 5 dermalen Naevi, 8 von 11 dysplastischen Naevi und 1 von 1 congenitalen Naevi.
4) Eine immunhistochemische Untersuchung normaler menschlicher kryopräservierter Gewebe ergab folgendes Ergebnis:
   Bei 3 untersuchten Hautproben waren sowohl das stratum basale, das stratum spinosum als auch die Melanozyten komplett negativ.
   Von den untersuchten endokrinen Organen zeigte bei 2 untersuchten Nebennieren weder die Cortex noch die Medulla eine Reaktion. Des weiteren waren 2 Goormaghtigh Zellproben, 4 Schilddrüsen und 4 Langerhans'sche Inseln negativ.
   Von den Geweben des Gastrointestinaltraktes waren die 3 untersuchten Schleimdrüsen, eine Zunge, zwei Speiseröhren, 3 Proben aus dem Zwölffingerdarm, 4 Gallenblasen, 4 Lebergewebe und 4 Pankreasgewebe komplett negativ. Im Dickdarm färbten sich bei den 4 untersuchten Proben die Becherzellen schwach an.
   Von den 2 untersuchten Lungengeweben waren beide negativ.
   Die Gewebe des Urogenitaltraktes waren komplett negativ, d.h. es wurden 2 Nieren, 2 Harnleiter, 1 Hoden, 1 Eierstock und 4 Brustgewebe untersucht.
   Die untersuchten lymphatischen Gewebe wie Milz (5 Proben) und Mandeln (1 Probe) waren ebenfalls negativ.
   Die untersuchten Skelett- und glatten Muskelgewebe, von denen je zwei Proben untersucht wurden, waren negativ. Die Blutgefäße waren ebenfalls negativ.
   Bindegewebe zeigte eine schwache Reaktion.
5) FACS-Analyse mit menschlichen peripheren Blutzellen (PBL) zeigte, daß der MAk mit ≈ 10 % der PBL reagierte.
6) Die Avidität, die in einem Festphasen-ELISA auf gereinigtem GD3 nach der bei Harlow E. and Lane D. (CSHL, p. 23, 1988) beschriebenen Methode bestimmt wurde, lag im Bereich von 2.3 x 10⁸ l/mol.
7) Der MAk lysiert die GD3 exprimierende SK-Mel28 Zelle in Gegenwart von humanem Serum (1:4 verdünnt) als Komplementquelle bis zu einer MAk-Konzentration von 1 µg/ml. Diese Versuche wurden in einem Zytotoxizitätstest entsprechend der Publikation von Welt, S., Carswell, E.A., Vogel, C.-W., Oettgen, H.F., Old, L.J. (Clin. Immunol. Immunopathol. 45:214-229, 1987) durchgeführt. Die Fähigkeit, humanes Komplement zu binden und hierdurch Tumorzellen zu töten, ist eine Eigenschaft, die auch andere MAk vom IgG3-Isotyp besitzen.

Die unter 1-7 beschriebenen Eigenschaften des MAk BW 2121 machen ihn den literaturbekannten MAks, insbesondere dem MAk R24 (Dippold, W.G., Lloyd, K.O., Li, L.T.C., Ikeda, H., Oettgen, H.F., Old, L.J., Proc. Natl. Acad. Sci. USA 77: 6144-6148, 1980) überlegen.

Der MAk BW2121 hat im allgemeinen folgende Vorteile gegenüber dem MAk R24:
1) weniger Kreuzreaktion mit Normalgewebe, d.h.:
   Der MAk R24 reagiert mit der Medulla der Nebenniere sowie mit den Goormaghtigh Zellen; Des weiteren reagiert er mit den Epithelien in den Mandeln und mit Bindegewebe
2) höhere Avidität:
   Der MAk R24 besitzt eine Avidität von 2 x 10⁷ l/mol, ≈ 1 log weniger als MAk BW 2121
3) besseres zytotoxisches Potential:
   Um 20 % Komplement-abhängige Zytolyse auf SK-Mel28 Melanomzellen zu erreichen, werden 10 µg/ml des MAk R24 benötigt, wohingegen 1 µg/ml des MAk BW 2121 den gleichen Effekt hat.

Die erfindungsgemäßen MAKs sind daher besonders geeignet in der in vitro oder in vivo Darstellung bzw. Therapie von Tumoren, welche das vom MAk BW 2121 erkannte Epitop ausprägen, insbesondere zum Nachweis von Melanomen und anderen GD3 und GQ1b exprimierenden Tumoren oder Geweben.

Das biochemisch, beispielsweise über Extraktion mit organischen Lösemitteln wie Chloroform-Methanol-Wasser-Gemische, isolierbare Antigen eignet sich besonders gut für die Herstellung bzw. Überprüfung von zum MAk BW 2121 äquivalenten Antikörpern oder immunologisch reaktiven Teilen davon und für die Herstellung von Mimetika.

Die erfindungsgemäßen Antikörper lassen sich mit radioaktiven Isotopen markieren, insbesondere mit Tc-99m (Schwarz, A., Steinstraesser, A., J. Nucl. Med. 28: 721, 1987). Eine weitere Möglichkeit stellt die Markierung mit paramagnetischen Verbindungen dar.
Des weiteren können die V-Gene der schweren und leichten Ketten des MAk BW 2121 nach der von Orlandi, R., Güssow, D., Jones, P.T., Winter, G. (Proc. Natl. Acad. Sci. USA 86: 3833-3837, 1989) beschriebenen Methode isoliert und nach der von Sanger, F., Nicklen, S., Coulson, A.R. (Proc. Natl. Acad. Sci. USA 74: 5463-5467, 1977) beschriebenen Methode die Nukleinsäuresequenz der essentiellen Bereiche des V-Gen Exons bestimmt werden. Die Nukleinsäuresequenzen und die dazugehörigen Aminosäuresequenzen sind in Tab. 1a und 1b dargestellt. Die klonierten V-Gene können ferner als chimäre MAk mit humanem verkürztem IgG3 Fc Teil (IgG3 ) und humanem C-Kappa in BHK-Zellen ausgeprägt werden (Wirth, M., Bode, J., Zettlmeissl, G., Hauser, H. Gene 73: 419-426, 1988). Des weiteren kann z.B. nach Polypeptidsynthese der CDRs bzw. von Teilen oder mehreren definierten CDRs die mrus (minimal recognition units) bestimmt und als wenig immunogene spezifische Peptide zu in vivo Lokalisation von Tumoren eingesetzt werden. Des weiteren kann nach der von Saragovi, H.U., Fitzpatrick, D., Raktabutr, A., Nakanishi, H., Kahn, M., Greene. M.I. (Science 253: 792-795, 1991) beschriebenen Methode mittels organischchemischer Synthese ein Mimetikum mit hoher Spezifität und Avidität gegen das von MAk BW 2121 definierte Epitop erzeugt werden. Die humanisierte V-Region des MAk BW 2121 kann rekombinant z.B. mit Nukleotidsequenzen verknüpft werden, die für ein Enzym oder eine Komplement-Komponente kodieren. Diese Konstrukte können wie in der deutschen Patentanmeldung P 41 06 389.9 (Ma 876) am Beispiel eines humanisierten αCEA MAk und der humanen β-Glucuronidase gezeigt, auf der Ebene der DNS verknüpft, als funktionelle Fusionsproteine exprimiert werden.

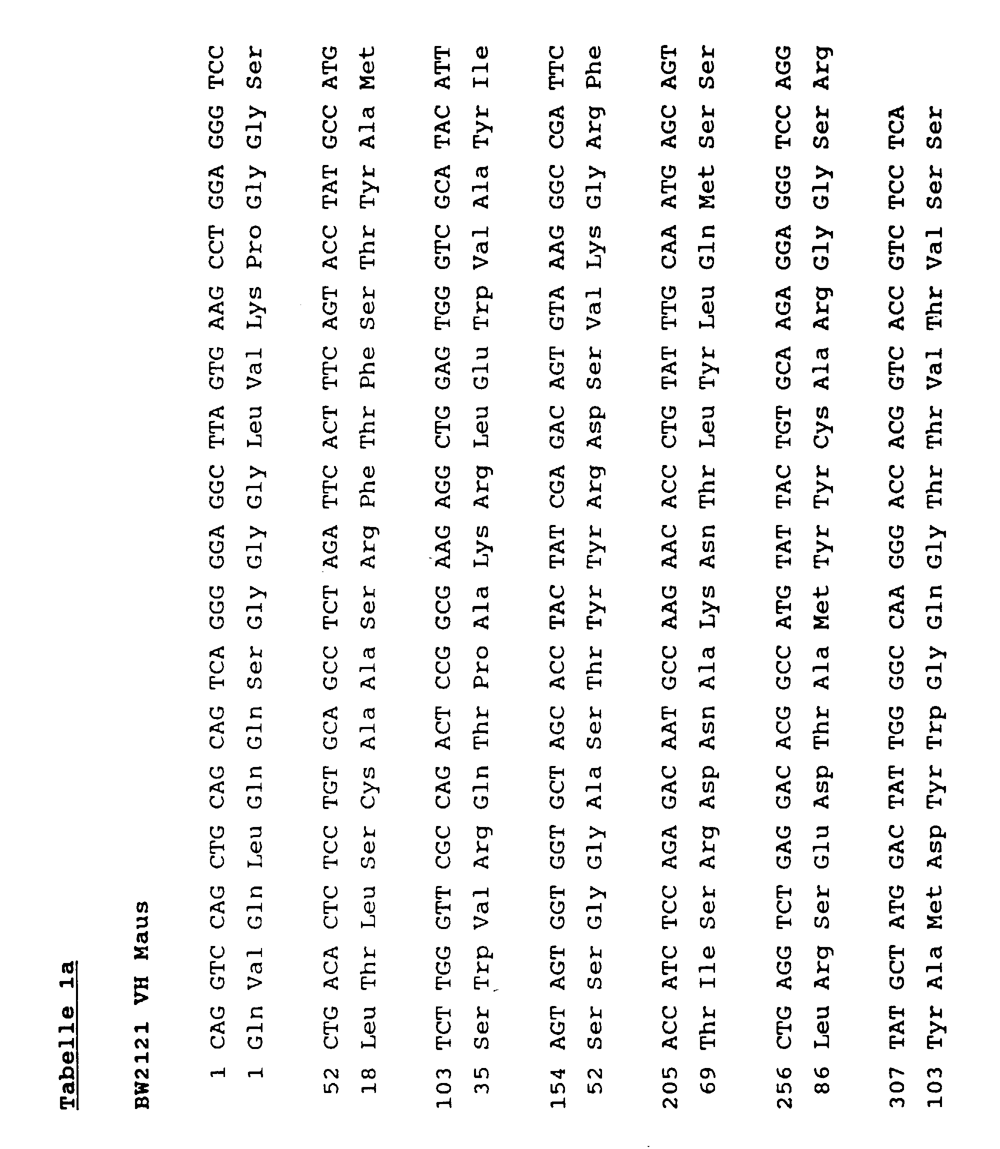

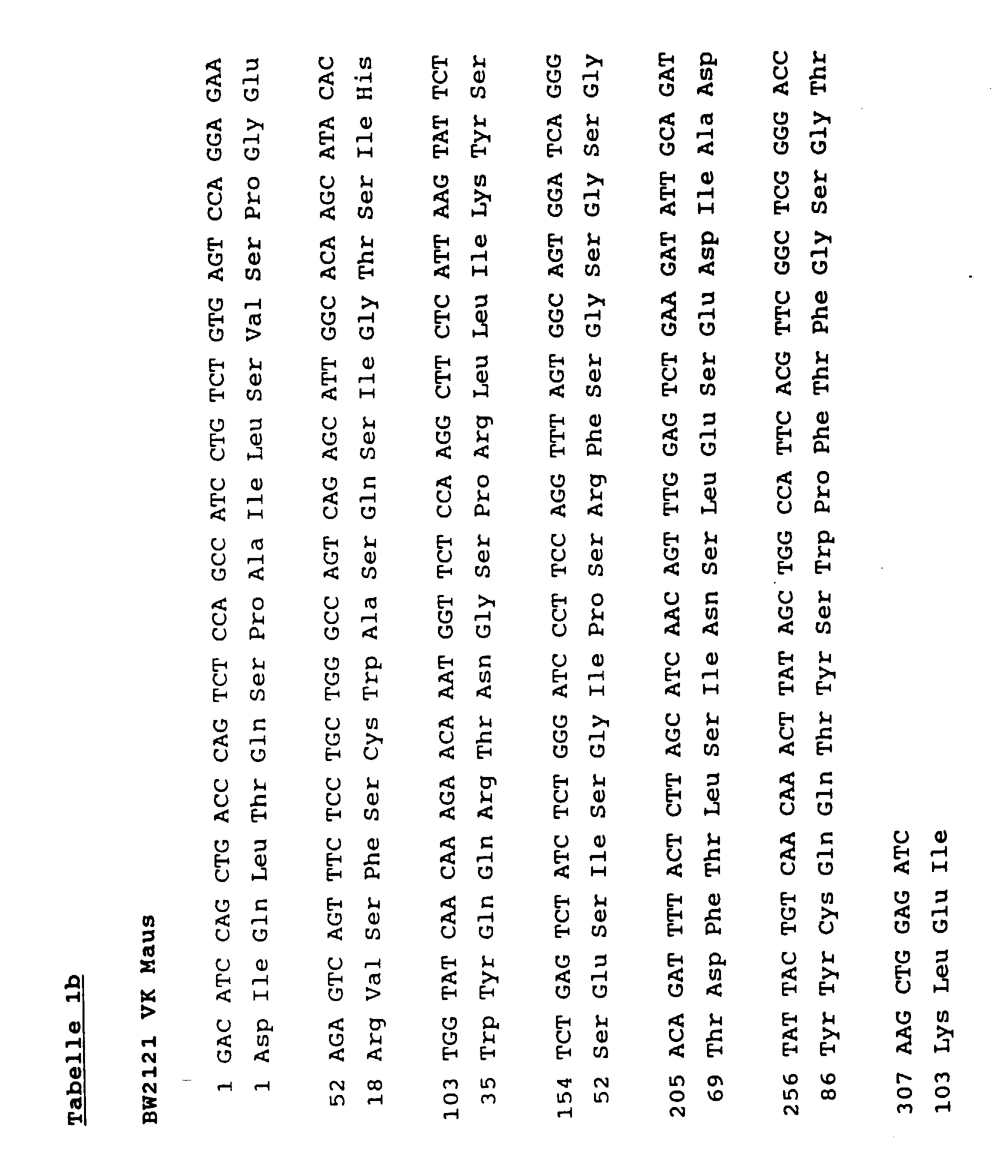

## Patentansprüche

1. Hybridom 2121 (DSM ACC 2036)

2. Monoklonaler Antikörper BW 2121, dadurch gekennzeichnet, daß er von dem Hybridom 2121(DSM ACC 2036) abstammt.

3. Monoklonale Antikörper oder Teile davon, dadurch gekennzeichnet, daß sie an ein Epitop, das von einem monoklonalen Antikörper nach Anspruch 2 erkannt wird, binden und immunhistochemisch nicht mit der Medulla der Nebenniere sowie mit den Goormaghtigh Zellen reagieren.

4. Monoklonale Antikörper und Teile davon nach Anspruch 3, dadurch gekennzeichnet, daß sie die V-Region des monoklonalen Antikörpers BW 2121, insbesondere die in den Tabellen 1a und 1b aufgeführten Aminosäureseqenzen enthalten.

5. Monoklonale Antikörper oder Teile davon nach Anspruch 3 oder 4, dadurch gekennzeichnet, daß der Antikörper ein chimärer, humanisierter, bi- oder oligospezifischer Antikörper ist, vorzugsweise ein humanisierter Antikörper.

6. Verfahren zur Herstellung eines monoklonalen Antikörpers nach mindestens einem der Ansprüche 3 - 5, dadurch gekennzeichnet, daß immunkompetente Säugetiere, wie beispielsweise die Maus, Ratte oder das Schaf, mit GD3- und/oder GQ1b-haltigem Immunogen immunisiert werden, die Immunzellen durch Fusion mit Myelomzellen immortalisiert werden und die GD3 und GQ1b spezifischen Hybridome isoliert werden.

7. Pharmazeutische Zusammensetzung, enthaltend einen oder mehrere monoklonale Antikörper nach mindestens einem der Ansprüche 2 - 5.

8. Verwendung von einem oder mehreren monoklonalen Antikörpern gemäß mindestens einem der Ansprüche 2 - 5 zur Herstellung eines Therapeutikums zur Behandlung von Tumoren, welche das vom MAK BW 2121 erkannte Epitop ausprägen.

9. Diagnostikum, enthaltend einen oder mehrere monoklonale Antikörper nach mindestens einem der Ansprüche 2 - 5.

10. Verwendung von einem oder mehreren monoklonalen Antikörpern gemäß mindestens einem der Ansprüche 2 - 5 zur Herstellung eines Diagnostikums zur in vitro oder in vivo Darstellung von Tumoren, welche das vom MAK BW 2121 erkannte Epitop ausprägen, insbesondere zum Nachweis von Melanomen und anderen GD3 und GQ1b exprimierenden Tumoren oder Geweben.

11. Fusionsproteine, dadurch gekennzeichnet, daß sie die V-Region des monoklonalen Antikörpers BW 2121, insbesondere die in den Tabellen 1a und 1b aufgeführten Aminosäuresequenzen, enthalten.

## Claims

1. The hybridoma 2121 (DSM ACC 2036)

2. The monoclonal antibody BW 2121, which is derived from the hybridoma 2121 (DSM ACC 2036).

3. A monoclonal antibody or part thereof, which binds to an epitope which is recognized by the monoclonal antibody as claimed in claim 2 and which does not react immunohistochemically with the adrenal medulla or with Goormaghtigh cells.

4. A monoclonal antibody and part thereof as claimed in claim 3, which contain the V region of the monoclonal antibody BW 2121, in particular the amino acid sequences listed in Tables 1a and 1b.

5. A monoclonal antibody or part thereof as claimed in claim 3 or 4, wherein the antibody is a chimeric, humanized, bispecific or oligospecific antibody, preferably a humanized antibody.

6. A process for preparing a monoclonal antibody as claimed in at least one of claims 3-5, which comprises immunizing immunocompetent mammals, such as the mouse, the rat or the sheep, with immunogen which contains GD3 and/or GQ1b, immortalizing the immune cells by fusion with myeloma cells, and isolating the hybridomas which are specific for GD3 and GQ1b.

7. A pharmaceutical composition, containing one or more monoclonal antibodies as claimed in at least one of claims 2-5.

8. The use of one or more monoclonal antibodies as claimed in at least one of claims 2-5 for preparing a therapeutic agent for treating tumors which express the epitope recognized by Mab BW 2121.

9. A diagnostic agent, containing one or more monoclonal antibodies as claimed in at least one of claims 2-5.

10. The use of one or more monoclonal antibodies as claimed in at least one of claims 2-5 for preparing a diagnostic agent for the in vitro or in vivo detection of tumors which express the epitope recognized by Mab BW 2121, in particular for detecting melanomas and other tumors or tissues which express GD3 and GQ1b.

11. A fusion protein, which contains the V region of the monoclonal antibody BW 2121, in particular the amino acid sequences listed in Tables 1a and 1b.

## Revendications

1. Hybridome 2121 (DSM ACC 2036).

2. Anticorps monoclonal BW 2121, caractérisé en ce qu'il dérive de l'hybridome 2121 (DSM ACC 2036).

3. Anticorps monoclonaux ou fragments de ceux-ci, caractérisés en ce qu'ils se lient à un épitope qui est reconnu par un anticorps monoclonal selon la revendication 2 et ne réagissent pas immunohistochimiquement avec la partie médullaire de la glande surrénale ni avec les cellules de Goormaghtigh.

4. Anticorps monoclonaux et fragments de ceux-ci selon la revendication 3, caractérisés en ce qu'ils contiennent la région V de l'anticorps monoclonal BW 2121, en particulier les séquences d'aminoacides indiquées dans les tableaux 1a et 1b.

5. Anticorps monoclonaux ou fragments de ceux-ci selon la revendication 3 ou 4, caractérisés en ce que l'anticorps est un anticorps chimère, humanisé, bi- ou oligospécifique, de préférence un anticorps humanisé.

6. Procédé pour la production d'un anticorps monoclonal selon au moins une des revendications 3 à 5, caractérisé en ce que des mammifères immunocompétents, comme par exemple la souris, le rat ou la chèvre, sont immunisés par un immunogène contenant GD3 et/ou GQ1b, les cellules immunes sont immortalisées par fusion avec des cellules de myélome et les hybridomes spécifiques de GD3 et GQ1b sont isolés.

7. Composition pharmaceutique, contenant un ou plusieurs anticorps monoclonaux selon au moins une des revendications 2 à 5.

8. Utilisation d'un ou de plusieurs anticorps selon au moins une des revendications 2 à 5, pour la préparation d'un agent thérapeutique destiné au traitement de tumeurs qui expriment l'épitope reconnu par l'AcM BW 2121.

9. Agent de diagnostic, contenant un ou plusieurs anticorps monoclonaux selon au moins une des revendications 2 à 5.

10. Utilisation d'un ou de plusieurs anticorps monoclonaux selon au moins une des revendications 2 à 5, pour la préparation d'un agent de diagnostic destiné à lavisualisation in vitro ou in vivo de tumeurs qui expriment l'épitope reconnu par l'AcM BW 2121, en particulier à la détection de mélanomes et d'autres tumeurs ou tissus exprimant GD3 et GQlb.

11. Protéines de fusion, caractérisées en ce qu'elles contiennent la région V de l'anticorps monoclonal BW 2121, en particulier les séquences d'aminoacides indiquées dans les tableaux 1a et 1b.
